# EUROPÄISCHE PATENTSCHRIFT

(11) **EP 4 140 419 B1**
(45) Veröffentlichungstag und Bekanntmachung des Hinweises auf die Patenterteilung: **30.08.2023**
(21) Anmeldenummer: 21192614.2
(22) Anmeldetag: 23.08.2021
(51) Int. Cl.: A61B 17/32, A61B 17/00

(54) **CHIRURGISCHES ULTRASCHALLGERÄT UND HANDSTÜCK DAFÜR**
ULTRASONIC SURGICAL APPARATUS AND HANDPIECE THEREFOR
APPAREIL CHIRURGICAL À ULTRASONS ET PIÈCE À MAIN ASSOCIÉ

(43) Veröffentlichungstag der Anmeldung: 01.03.2023
(73) Patentinhaber: SRA Developments Ltd., Bremridge Ashburton, Devon TQ13 7JX (GB)
(72) Erfinder: Besch, Hansjörg, 72810 Gomaringen (DE); Faßnacht, Malte, 72070 Tübingen (DE); Graham, Chris, Paignton, Devon TQ3 2LR (GB)
(74) Vertreter: Schneider, Peter Christian

(56) Entgegenhaltungen:
- EP-B1- 2 366 345
- DE-T2- 60 132 690
- US-A1- 2013 289 591
- US-A1- 2018 296 238

## Beschreibung

Die Erfindung bezieht sich auf ein Handstück für ein chirurgisches Ultraschallgerät, umfassend ein Gehäuse, in welches eine Funktionseinheit, die einen Ultraschallwandler und ein ultraschallübertragend mit diesem verbundenes Instrument aufweist, derart einsetzbar ist, dass sie das Gehäuse entlang einer Längsachse durchsetzt und um die Längsachse rotierbar in dem Gehäuse gelagert und über eine Steuerleitung mit einem Schalter des Gehäuses elektrisch verbunden ist,
wobei die Steuerleitung eine drehentkoppelnde, elektrische Schnittstelle aufweist, umfassend eine gehäuseseitige Platine und eine funktionseinheitsseitige Platine, von denen die eine einen zur Längsachse koaxialen Ringkontakt und die andere einen den Ringkontakt elastisch kontaktierenden, axial erstreckten Kontaktstift trägt.

Die Erfindung bezieht sich weiter auf ein chirurgisches Ultraschallgerät, umfassend
- eine Funktionseinheit, die einen Ultraschallwandler und ein ultraschallübertragend mit diesem verbundenes Instrument sowie ein mit einer externen Versorgungs- und Steuereinheit verbindbares Versorgungs- und Steuerkabel aufweist,
- ein Handstück mit einem Gehäuse, in welches die Funktionseinheit derart eingesetzt ist, dass sie das Gehäuse entlang einer Längsachse durchsetzt und um die Längsachse rotierbar in dem Gehäuse gelagert ist,
wobei ein Steuerstrang des Versorgungs- und Steuerkabels über eine Steuerleitung mit einem Schalter des Gehäuses elektrisch verbunden ist, und die Steuerleitung eine drehentkoppelnde, elektrische Schnittstelle aufweist, umfassend eine gehäuseseitige Platine und eine funktionseinheitsseitige Platine, von denen die eine einen zur Längsachse koaxialen Ringkontakt und die andere einen den Ringkontakt elastisch kontaktierenden, axial erstreckten Kontaktstift trägt.

### Stand der Technik

Derartige chirurgische Ultraschallgeräte und Handstücke dafür sind bekannt aus der DE 601 32 690 T2.

Chirurgische Ultraschallgeräte sind in der modernen Chirurgie in einer Vielzahl von Ausführungsformen weit verbreitet. Gemeinsam ist ihnen, dass ein chirurgisches Instrument, beispielsweise ein Skalpell, eine Zange oder ähnliches mittels eines ultraschallleitend angeschlossenen Ultraschallwandlers in hochfrequente Schwingungen geringer Amplitude versetzbar ist. Die medizinischen Vorteile und Wirkungen dieses Ansatzes sind dem Fachmann bekannt und nicht Bestandteil der vorliegenden Erfindung, sodass nicht näher auf sie eingegangen werden soll.

Technisch herausfordernd ist die handliche Gestaltung des Ultraschallwandlers und sein ultraschallleitender Anschluss an das Instrument, welches selbst möglichst wenig von herkömmlichen, d.h. nicht Ultraschall-unterstützten Instrumenten verschieden sein soll. Es hat sich bewährt, das Instrument, den Ultraschallwandler und ihre ultraschallleitende Verbindung, oft ausgebildet als ein häufig als Wellenleiter bezeichneter Schallleiterstab, in einer kompakten Funktionseinheit zusammenzufassen. Diese Funktionseinheit umfasst regelmäßig auch ein Versorgungs- und Steuerkabel, welches an eine externe Versorgungs- und Steuereinheit anschließbar ist. Insbesondere ist ein Versorgungsstrang des Versorgungs- und Steuerkabels mit dem Ultraschallwandler verbunden und beaufschlagt diesen mit einer elektrischen Leistung aus dem Versorgungstrakt, typischerweise einer Hochfrequenz-Spannungs- oder Stromquelle, der externen Versorgungs- und Steuereinheit. Allerdings darf der Ultraschallwandler nicht im Dauerbetrieb arbeiten. Insbesondere muss es dem Chirurgen möglich sein, das Instrument gezielt mit Ultraschallschwingungen anzuregen und vorzugsweise diese auch in ihrer Leistung zu variieren. Hierzu ist regelmäßig ein vom Chirurgen betätigbarer Schalter vorgesehen. Besonders günstig ist es dabei, wenn der Schalter von der das Ultraschallgerät haltenden Hand selbst betätigt werden kann. Ein solcher Schalter ist über eine Steuerleitung mit einem Steuerstrang des Versorgungs- und Steuerkabels verbunden, der seinerseits zu einem Steuertrakt der Versorgungs- und Steuereinheit führt. Am Schalter gegebene Steuerbefehle, beispielsweise "an" und "aus" bzw. konkrete Leistungsvorgaben, werden also über die Steuerleitung zum Steuerstrang des Kabels und über dieses zum Steuertrakt der externen Versorgungs- und Steuereinheit geleitet, die dann ihrerseits deren Versorgungstrakt entsprechend ansteuert, sodass diese den Ultraschallwandler mit entsprechender elektrischer Leistung versorgt.

Ein solches Gerät ist in der eingangs genannten, gattungsbildenden Druckschrift beschrieben. Dort wird insbesondere auf das Problem aufmerksam gemacht, dass ein Schalter, der an einer bestimmten Stelle der Funktionseinheit selbst fixiert ist, bei der Handhabung des Gerätes, insbesondere beim korrekten Ansetzen des Instrumentes, im Wege sein kann. Die Druckschrift schlägt daher vor, den Schalter an einem gesonderten Handstück vorzusehen, welches um die Funktionseinheit rotierbar gelagert ist, sodass der Schalter stets in eine für die vom Chirurgen beabsichtigte Handhabung günstige Position gedreht werden kann. Problematisch ist dabei jedoch die Ausgestaltung der Steuerleitung, die einerseits eine elektrische Leitung zwischen dem Schalter und dem Steuerstrang des Versorgungs- und Steuerkabels herstellen muss, andererseits aber auch eine Drehentkopplung zwischen dem den Schalter beherbergenden Handstück und dem mit der Funktionseinheit verbundenen Versorgungs- und Steuerkabel bereitstellen muss. Bei der vorbekannten Gestaltung besteht die Funktionseinheit aus einem Hauptteil, der den Ultraschallwandler enthält und das Versorgungs- und Steuerkabel trägt, und dem Instrument, welches über einen Schaft mit dem Hauptteil verschraubbar ist. Der Schaft durchsetzt das den Schalter tragende Handstück in axialer Richtung. Das Handstück ist rotierbar um den Schaft bzw. der Schaft rotierbar im Handstück gelagert. Eine nach vorne, zum Instrument hin gerichtete Stirnseite des Funktionseinheits-Hauptteils trägt zwei konzentrische Ringkontakte, die über im Funktionseinheits-Hauptteil geführte Verbindungsleitungen mit dem Steuerstrang des Versorgungs- und Steuerkabels verbunden sind. Eine korrespondierende, nach hinten, zum Funktionseinheits-Hauptteil hin gerichtete Stirnseite des Handstücks trägt zwei konzentrische Ringe von axial ausgerichteten Kontaktstiften, die einerseits über Handstück-interne Kabel mit dem Schalter verbunden sind und die andererseits im Zusammenbauzustand auf den Ringkontakten des Funktionseinheits-Hauptteils aufsetzen. Der dadurch entstehende elektrische Kontakt zwischen dem Schalter und dem Steuerstrang des Versorgungs- und Steuerkabels bleibt auch bei Rotation des Handstücks um die gemeinsame Symmetrieachse der Ringkontakte einerseits und der Kontaktstift-Ringe andererseits erhalten. Um auch Abrieb und Fertigungstoleranzen ausgleichen zu können, sind die Kontaktstifte elastisch ausgebildet.

Zur technischen Umsetzung dieses im Prinzip sinnvollen Ansatzes verhält sich die zitierte Druckschrift nicht näher. Insbesondere sind keine konkreten Angaben zur elastischen Ausgestaltung der Kontaktstifte gemacht. Diese stellt sich für den Fachmann in der Tat auch recht schwierig dar. Kostengünstige und effektive Kontaktstifte sind bevorzugt aus metallischem Werkstoff gefertigt. Dieser hat aber üblicherweise nicht die erforderliche Elastizität. Elastomere Kontaktstifte hingegen sind nur durch hohen technischen Aufwand und unter erheblichen Kosten mit der erforderlichen elektrischen Leitfähigkeit ausstattbar. Diese Diskrepanz bleibt in der genannten Druckschrift ungelöst.

Ein alternativer Ansatz zur Kontaktierung ist in den Druckschriften EP 2 366 345 B1, US 2018/0296238 A1 und US 2013/0289591 A1 vorgeschlagen. Hier wird eine in radialer Richtung wirkende elektrische Verbindung vorgeschlagen, bei der einander in radialer Richtung entgegengesetzt federvorgespannte Kontaktelemente miteinander wechselwirken, wobei der Rotationsfreiheitsgrad um die Längsachse erhalten bleibt. Bei einer Ausgestaltung gemäß EP 2 366 345 B1 oder US 2013/0289591 A1 finden offene, nach radial außen vorgespannter Klemm-, Schleif- und Kontaktringe Verwendung; die Ausgestaltung gemäß US 2018/0296238 A1 setzt ebenfalls offene, jedoch nach radial innen federvorgespannte Klemm-, Schleif- und Kontaktringe ein. Zur Montage, die, um zügig und zuverlässig vonstattengehen zu können, auf geführte, rein axiale Relativbewegungen von miteinander zu verbindenden Elementen beschränkbar sein sollte, sind aber komplizierte mechanischen Konstruktionen mit Anlaufschrägen erforderlich, die ein temporäres Auf- bzw. Zubiegen der Ringe während des Einschiebens des Kontakt-Gegenstücks ermöglichen. Bei den im Bereich der Kunststoff-Spritzgusstechnik üblichen Fertigungstoleranzen muss dieser Ansatz, der leicht zu Verkantungen und einem Abscheren von Kontaktelementen Führen kann, als heikel angesehen werden. Als günstiger wird daher allgemein der zuvor erläuterte Ansatz einer rein axial wirkenden Kontaktierung angesehen.

### Aufgabenstellung

Es ist die Aufgabe der vorliegenden Erfindung, eine technisch und unter Kostenaspekten günstig realisierbare Ausführungsform des zuvor geschilderten, grundsätzlich positiven Ansatzes zur Verfügung zu stellen.

### Darlegung der Erfindung

Diese Aufgabe wird in Verbindung mit den Merkmalen des Oberbegriffs von Anspruch 1 dadurch gelöst, dass der Kontaktstift als steifer Metallstift ausgebildet ist, der axial beweglich gelagert und in Richtung auf die Ringkontakte federvorgespannt ist.

Die Erfindung wird in Verbindung mit den Merkmalen des Oberbegriffs von Anspruch 11 dadurch gelöst, dass der Kontaktstift als steifer Metallstift ausgebildet ist, der axial beweglich gelagert und in Richtung auf die Ringkontakte federvorgespannt ist.

Bevorzugte Ausführungsformen sind Gegenstand der abhängigen Patentansprüche.

Die Grundidee der Erfindung ist es, von den Vorgaben des Standes der Technik nach einer Zusammenführung eigentlich unvereinbarer Eigenschaften in einem Bauteil abzurücken und stattdessen mehrere Bauteile zu verwenden, diese jedoch jeweils im Hinblick auf eine ihnen speziell zugedachte Teilfunktion hin zu optimieren. Wie oben bereits erwähnt, ist Metall, insbesondere Messing, Gold oder Kupfer, eine optimale Materialwahl im Hinblick auf elektrische Leitfähigkeit. Ein steifer Stift aus solchem Material ist zudem ausgesprochen robust und langlebig. Die zur Aufrechterhaltung des elektrischen Kontaktes auch bei Abrieb und/oder Fertigungstoleranzen erforderliche Elastizität wird hingegen durch ein zusätzliches Element, nämlich eine Feder realisiert, wobei diese Feder insbesondere auf ihre Elastizitätseigenschaften hin optimiert sein kann. Die federvorgespannte, axial bewegliche Lagerung des Kontaktstiftes schließlich führt zu einer elektrisch zuverlässigen, robusten Kontakteinheit, die in der Lage ist, auch große Toleranzen zu überbrücken. Dies ist insbesondere von Vorteil, wenn das Handstück, in dem sich die erfindungszentrale Schnittstelle befindet, als kostengünstiges Einmal-Produkt gefertigt wird, beispielsweise im Wesentlichen aus Kunststoff im Spritzgussverfahren. Bei derartigen Produkten lassen sich enge Toleranzen nämlich nur mit unwirtschaftlich hohem Aufwand sicherstellen. Mit dem erfindungsgemäßen Ansatz jedoch können kostengünstig hergestellte Einmal-Handstücke verwendet werden, in welche hochpräzise gefertigte, kostspielige und wiederverwendbare Funktionseinheiten einsetzbar sind.

Günstigerweise ist die funktionseinheitsseitige Platine an einem um die Längsachse rotierbar im Gehäuse gelagerten Funktionseinheits-Adapter fixiert, in welchen die Funktionseinheit reversibel, drehfest und mit elektrischem Kontakt zu der funktionseinheitsseitigen Platine einsetzbar, insbesondere verrastbar ist. In Bezug auf das erfindungsgemäße Ultraschallgerät, welches das Handstück und eine darin eingesetzte Funktionseinheit umfasst, bedeutet dies, dass die funktionseinheitsseitige Platine an einem um die Längsachse rotierbar im Gehäuse gelagerten Funktionseinheits-Adapter fixiert ist, in welchem die Funktionseinheit reversibel, drehfest und mit elektrischem Kontakt zu der funktionseinheitsseitigen Platine fixiert, insbesondere verrastet ist. Auch dieser Ansatz ist dem bevorzugten Einsatz von kostengünstigen Kunststoff-Spritzgussverfahren geschuldet. Diese lassen nämlich nur eine beschränkte Formkomplexität zu. Es hat sich daher als günstig erwiesen, ein zusätzliches Bauteil, nämlich den Funktionseinheits-Adapter zu schaffen, der im Hinblick auf eine vorzugsweise verrastende Fixierung der Funktionseinheit hin optimiert ist. Beispielsweise kann er grob hohlzylindrisch mit nach radial innen wirkenden Rastelementen, die mit korrespondierenden Rastelementen der Funktionseinheit wechselwirken können, ausgestaltet sein. Nach außen hin bedarf es lediglich einer abschnittsweisen Radialsymmetrie, um eine geeignete, mechanische Lagerschnittstelle zu einfach gestalteten Gegenstrukturen des Gehäuses bereitzustellen.

Dabei kann der Funktionseinheits-Adapter, wie bevorzugt vorgesehen, wenigstens einen mit der funktionseinheitsseitigen Platine elektrisch verbundenen Rastkontakt aufweisen, der räumlich mit einem Gegenkontakt der Funktionseinheit derart korrespondiert, dass der Rastkontakt und der Gegenkontakt im eingesetzten Zustand der Funktionseinheit einander elektrisch kontaktieren. Im Kontext des erfindungsgemäßen Ultraschallgerätes bedeutet dies, dass der Funktionseinheits-Adapter wenigstens einen mit der funktionseinheitsseitigen Platine elektrisch verbundenen Rastkontakt aufweist, der räumlich mit einem Gegenkontakt der Funktionseinheit derart korrespondiert, dass der Rastkontakt und der Gegenkontakt einander elektrisch kontaktieren. Der Rastkontakt kann, wie bevorzugt vorgesehen, in das Kunststoffmaterial des Funktionseinheits-Adapters eingespritzt sein. Auch die Verbindung zur funktionseinheitsseitigen Platine kann in das Material des Adapters eingespritzt sein. Denkbar ist aber auch eine separate Leitungsführung durch Kabel oder flexible Leiterbahnen außerhalb des Adapters. Die funktionseinheitsseitige Platine und der Funktionseinheits-Adapter können, müssen aber nicht einstückig ausgebildet sein. Im Gegenteil erscheint es günstiger, die funktionseinheitsseitige Platine als separates und im Hinblick auf ihre Platinenfunktion hin optimiertes Bauteil auszubilden, welches beispielsweise ringförmig auf den Funktionseinheits-Adapter aufgeschoben und mit diesem verrastet werden kann.

Für die gehäuseseitige Platine hat es sich als günstig erwiesen, diese nicht unmittelbar im Gehäuse zu fixieren, sondern formschlüssig in einem im Gehäuse fixierten Platinenadapter. In diesem ist sie vorzugsweise verrastet. Die zur zuverlässigen Direkt-Verrastung mit dem Gehäuse erforderliche Formgebung der Platine verträgt sich schlecht mit den Eigenschaften von für Platinen geeigneten Materialien (z.B. elektrische Durchschlagsfestigkeit). Bei der bevorzugten, mittelbaren Fixierung der Platine im Gehäuse über besagten Platinenadapter können diesem jedoch die notwenige Formkomplexität auferlegt und die Platine selbst einfach geformt werden, sodass insgesamt eine sichere Fixierung und eine im Hinblick auf die jeweiligen Spezialfunktionen optimierte Ausgestaltung der einzelnen Bauteile erreicht werden kann. Der Platinenadapter selbst kann vorzugsweise ebenfalls formschlüssig, insbesondere durch Verrastung im Gehäuse fixiert sein.

Bei der Verwendung eines Platinenadapters kann diesem, wie bevorzugt vorgesehen, eine weitere Funktion zugewiesen werden. So ist günstigerweise vorgesehen, dass der Kontaktstift in einem achsparallel zur Längsachse erstreckten Kanal des aus einem elektrisch isolierenden Material gefertigten Platinenadapters angeordnet ist. Dies bedeutet zunächst, dass, wie allgemein bevorzugt vorgesehen, der Kontaktstift auf der gehäuseseitigen Platine angeordnet ist. Der Ringkontakt ist in diesem Fall entsprechend auf der funktionsseitigen Platine angeordnet ist. Grundsätzlich ist zwar auch eine umgekehrte oder im Fall von mehreren Paaren von Ringkontakten und Kontaktstiften sogar eine "gemischte" Zuordnung möglich. Im Hinblick auf die dadurch mögliche Doppelfunktion des Platinenadapters, nämlich einerseits zur Fixierung der gehäuseseitigen Platine und andererseits zur Lagerung des bzw. der Kontaktstifte, wird jedoch die erstere Variante bevorzugt.

Die Lagerung des Kontaktstiftes im Platinenadapter kann unmittelbar oder mittelbar erfolgen. Letzteres ist im Hinblick auf den damit verbundenen Montageaufwand günstiger. Allgemein hat es sich nämlich als günstig erwiesen, wenn jeder Kontaktstift Bestandteil einer Kontakt-Baueinheit ist, die ein elektrisch mit der zugeordneten Platine verbundenes Kontaktgehäuse und einen eine axiale Stirnwand des Kontaktgehäuses durchsetzenden, innerhalb des Kontaktgehäuses mittels einer Feder vorgespannten und als der Kontaktstift wirkenden Stößel umfasst, wobei zumindest das Kontaktgehäuse und der Stößel aus einem elektrisch leitfähigen Material gefertigt sind. Derartige Kontakt-Baueinheiten lassen sich separat fertigen, auf der Platine festlöten und gegebenenfalls beim Einsetzen der Platine in einen Platinenadapter in Axialkanäle des letzteren einsetzen, sodass eine seitliche, elektrisch isolierende Umhausung gegeben ist. Der Fachmann wird verstehen, dass die Stößel zumindest bereichsweise aus besagten Kanälen des Platinenadapters hervorragen müssen, um den korrespondierenden Ringkontakt kontaktieren zu können.

Insbesondere in Fällen, in denen die Funktionseinheit ein zangenartiges Instrument umfasst, hat es sich als praktisch erwiesen, wenn das Gehäuse pistolengriffartig mit einem den Schalter tragenden Griffstück und einem endständig an dem Griffstück in einem Winkel dazu angeordneten Einsatzstück zur Aufnahme der Funktionseinheit ausgebildet ist. Besonders vorteilhaft ist es dabei, wenn das Griffstück und das Einsatzstück gemeinsam einteilig ausgebildet sind, um ein aus möglichst wenigen Komponenten bestehendes, günstig herstellbares und daher zum Einmal-Gebrauch besonders geeignetes Handstück zu schaffen. Das Griffstück kann dann noch weitere Betätigungselemente, wie beispielsweise einen Hebel umfassen, der in geeigneter Weise mechanisch mit der Greifmechanik des Instrumentes gekoppelt ist.

Bislang war der Einfachheit halber stets lediglich von einem Ringkontakt und einem zugehörigen Kontaktstift die Rede. Bei einer Weiterbildung der Erfindung ist jedoch vorgesehen, dass der Schalter mehrere, insbesondere unabhängig voneinander betätigbare, Schaltelemente und/oder das Handstück mehrere Schalter aufweist und die drehentkoppelnde, elektrische Schnittstelle eine entsprechende Mehrzahl von Paaren aus je einem Ringkontakt und einem oder mehreren, korrespondierenden Kontaktstiften aufweist, wobei jedes der Paare mit einem anderen oder einer anderen Kombination der Schalter oder Schaltelemente elektrisch verbunden ist. Mit anderen Worten kann die erfindungsgemäß gestaltete Schnittstelle mehrere elektrische Kanäle aufweisen, über die unterschiedliche und nahezu beliebig komplexe Steuersignale übermittelt werden können. Beispielsweise kann über einen Kanal die Aktivierung bzw. Deaktivierung der Ultraschallanregung des Instrumentes und über einen anderen Kanal die Anregungsleistung gesteuert werden.

Weitere Einzelheiten und Vorteile der Erfindung ergeben sich aus der nachfolgenden speziellen Beschreibung und den Zeichnungen.

### Kurzbeschreibung der Zeichnungen

Es zeigen:
- Figur 1:: eine teilweise geschnittene Übersichtsdarstellung eines erfindungsgemäßen Ultraschallgerätes,
- Figur 2:: den Funktionseinheits-Adapter samt elektrischer Schnittstelle des Gerätes von Figur 1,
- Figur 3:: die Platinen der elektrischen Schnittstelle von Figur sowie
- Figur 4:: die Kontakteinheit der gehäuseseitigen Schnittstelle von Figur 3 in zwei Funktionsstellungen.

### Beschreibung bevorzugter Ausführungsformen

Gleiche Bezugszeichen in den Figuren deuten auf gleiche oder analoge Elemente hin.

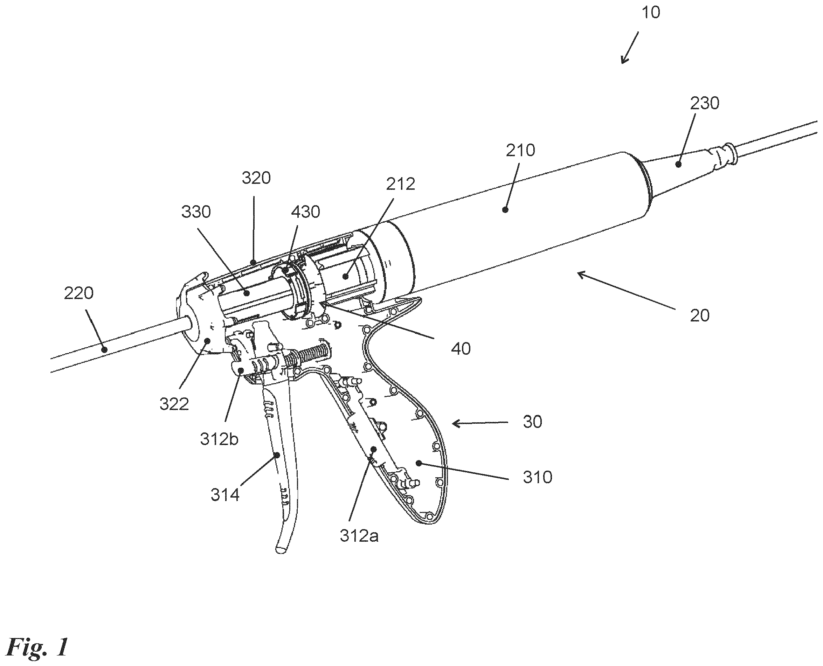

Figur 1 zeigt eine teilweise geschnittene Übersichtsdarstellung eines erfindungsgemäßen chirurgischen Ultraschallgerätes 10. Das Ultraschallgerät 10 umfasst im Wesentlichen zwei Bestandteile, nämlich eine Funktionseinheit 20 und ein Handstück 30. Die Funktionseinheit 20 weist einen Ultraschallwandler 210 auf, der an seinem distalen Ende über einen Schaft 220 vibrationsübertragend mit einem nicht dargestellten chirurgischen Instrument am distalen Ende des Schaftes 220 verbunden ist. An seinem proximalen Ende ist der Ultraschallwandler 210 mit einem Versorgungs- und Steuerkabel 230 verbunden. Ein nicht im Detail dargestellter Versorgungsstrang des Versorgungs- und Steuerkabels 230 ist an seinem distalen Ende derart an den Ultraschallwandler 210 angeschlossen, dass dessen vibrationserzeugende Elemente, beispielsweise Stapel von Piezokristallen, aus einer am proximalen Ende des Versorgungsstranges angeschlossenen Hochfrequenzquelle eines nicht dargestellten Generators mit elektrischer Leistung beaufschlagt werden können. Ein ebenfalls nicht im Detail dargestellter Steuerstrang des Versorgungs- und Steuerkabels 230 ist hingegen an den vibrationserzeugenden Einheiten des Ultraschallwandlers 210 vorbei in den Bereich eines das distale Ende des Ultraschallwandlers 210 bildenden Raststutzens 212 geführt, wie weiter unten noch näher erläutert wird. Das proximale Ende des Steuerstrangs kann mit dem Steuertrakt des Generators verbunden werden, um Steuerungsbefehle zu senden bzw. zu empfangen und die Hochfrequenzquelle entsprechend anzusteuern.

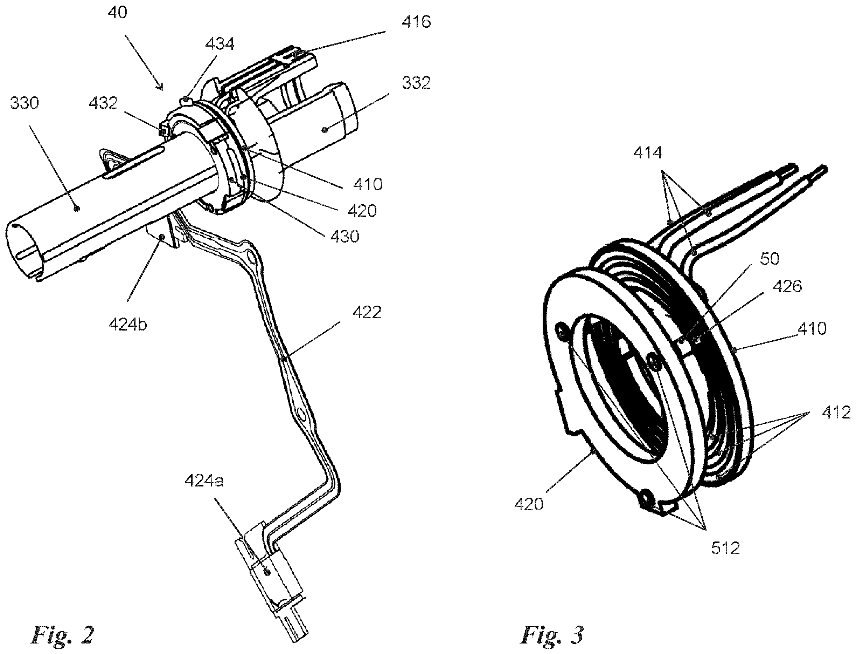

In der in Figur 1 gezeigten Konstellation ist die Funktionseinheit 20 in das Handstück 30 eingesetzt. Bei der dargestellten Ausführungsform ist das Handstück 30 pistolengriffartig ausgebildet und umfasst ein Griffstück 310 und ein in einem Winkel von ca. 60° dazu angeordnetes Einsatzstück 320. Bei der gezeigten Ausführungsform sind Griffstück 310 und Einsatzstück 320 einteilig, d. hDie Funktionseinheit 20 ist in einer das Einsatzstück 320 axial durchsetzenden Weise in dieses eingesetzt. Insbesondere weist das Einsatzstück 320 einen Funktionseinheits-Adapter 330 auf, in welchem die Funktionseinheit 20 reversibel fixiert, insbesondere verrastet ist, wie weiter unten im Kontext von Figur 2 noch näher beschrieben werden soll. Der Funktionseinheits-Adapter 330 ist um die Längsachse des Einsatzstücks 320 rotierbar in diesem gelagert. Mittels eines Drehrades 322 lässt sich die relative Drehlage zwischen dem Funktionseinheits-Adapter 330 und dem Einsatzstück 320 variieren.

Das Griffstück 310 trägt zwei Drucktasten 312a,b, die im Montageendzustand auf in Figur 2 erkennbare Schalter 424a,b wirken, welche ihrerseits in weiter unten näher beschriebener Weise elektrisch mit dem Steuerstrang des Versorgungs- und Steuerkabels 230 verbunden sind, sodass durch Betätigung der Drucktasten 312a,b bzw. der Schalter 424a,b Steuerbefehle für den Generator erzeugt werden können. Beispielsweise können der eine Schalter 424a bzw. die zugeordnete Drucktaste 312a zur Umschaltung zwischen unterschiedlichen Leistungsniveaus und der andere Schalter 424b bzw. die zugeordnete Drucktaste 312b zur grundsätzlichen Aktivierung bzw. Deaktivierung der Ultraschallanregung des Instrumentes genutzt werden. Details der genannten, in Figur 1 nicht dargestellten elektrischen Verbindung stehen im Fokus der vorliegenden Erfindung und sollen weiter unten noch näher erläutert werden. In Figur 1 ist lediglich grob die drehentkoppelnde elektrische Schnittstelle 40 dargestellt, auf die weiter unten noch im Detail eingegangen wird.

Bei der dargestellten Ausführungsform umfasst das Griffstück 310 zusätzlich einen Hebel 314, der in einer nicht im Detail dargestellten Weise mechanisch mit dem chirurgischen Instrument am distalen Ende des Schaftes 220 gekoppelt ist, sodass durch Betätigung des Hebels mechanische Verstellungen am Instrument vorgenommen werden können. Beispielsweise kann es sich bei dem chirurgischen Instrument um eine Zange handeln, deren Backen mittels des Hebels 314 geöffnet und geschlossen werden können.

Figur 2 zeigt in vergrößerter Darstellung den Funktionseinheits-Adapter 330 und die von diesem axial durchsetzte Schnittstelle 40. Details der Schnittstelle 40 sind in Figur 3 vergrößert dargestellt. Die wesentlichen elektrischen Elemente der Schnittstelle 40 sind deren funktionseinheitsseitige Platine 410 und ihre gehäuseseitige Platine 420. Diese sind als im Wesentlichen parallele Ringplatinen ausgebildet. Bei der dargestellten Ausführungsform trägt die funktionseinheitsseitige Platine 420 drei konzentrische Ringkontakte 412, die über Anschlusskabel 414 mit Rastkontakten 416 auf einem von mehreren Rasthaken 332 am proximalen Ende des Funktionseinheits-Adapters 330 elektrisch verbunden sind. Die funktionseinheitsseitige Platine 410 ist sowohl drehfest als auch axial fest auf dem Funktionseinheits-Adapter 330 fixiert.

Beim Einsetzen der Funktionseinheit 20 in das Handstück 30, insbesondere in dessen Einsatzstück 320, insbesondere in den in diesem gelagerten Funktionseinheits-Adapter 330 wird der Schaft 220 durch den hohl ausgebildeten Funktionseinheits-Adapter 330 geschoben, bis der Raststutzen 212 am distalen Ende des Ultraschallwandlers 210 in den Bereich der Rasthaken 332 gelangt und aufgrund seiner mit dieser korrespondierenden Formgebung mechanisch verrastet. Hierdurch wird eine sowohl axial als auch gegen Relativverdrehung feste, gleichwohl reversible Verbindung zwischen der Funktionseinheit 20 und dem Funktionseinheits-Adapter 330 geschaffen. Der Raststutzen 212 weist nicht im Detail dargestellte Gegenkontakte zu den Rastkontakten 416 auf, sodass bei der mechanischen Verrastung zugleich ein elektrischer Kontakt zwischen dem Steuerstrang des Versorgungs- und Steuerkabels 230 mit der funktionseinheitsseitigen Platine 410, insbesondere mit deren Ringkontakten 412 zustande kommt.

Ein weiterer Bestandteil der Schnittstelle 40 ist die gehäuseseitige Platine 420. Diese ist über einen Platinenadapter 430 axial und drehfest mit dem Gehäuse des Handstücks 30, insbesondere mit dessen Einsatzstück 320 verbunden. Bei der dargestellten Ausführungsform erfolgt diese Verbindung als Rastverbindung, wozu der Platinenadapter 430 einerseits nach innen wirkende Rastnasen 432 zur Fixierung der gehäuseseitigen Platine 420 und andererseits nach außen wirkende Rastnasen 434 zu seiner eigenen Fixierung im Einsatzstück 320 aufweist. Die gehäuseseitige Platine 420 ist über eine flexible Leiterbahn 422 mit den Schaltern 424a,b verbunden, die ihrerseits mittels der Drucktasten 312a,b im Griffstück 310 betätigbar sind. Die Details der unmittelbaren Kontaktierung der gehäuseseitigen Platine 420 durch die flexible Leiterbahn 422 ist der besseren Übersichtlichkeit halber in Figur 2 nicht dargestellt. Der Fachmann wird jedoch verstehen, wie eine elektrische Kontaktierung insbesondere von mehreren Lötstellen 512 auf der gehäuseseitigen Platine 420, zu erfolgen hat.

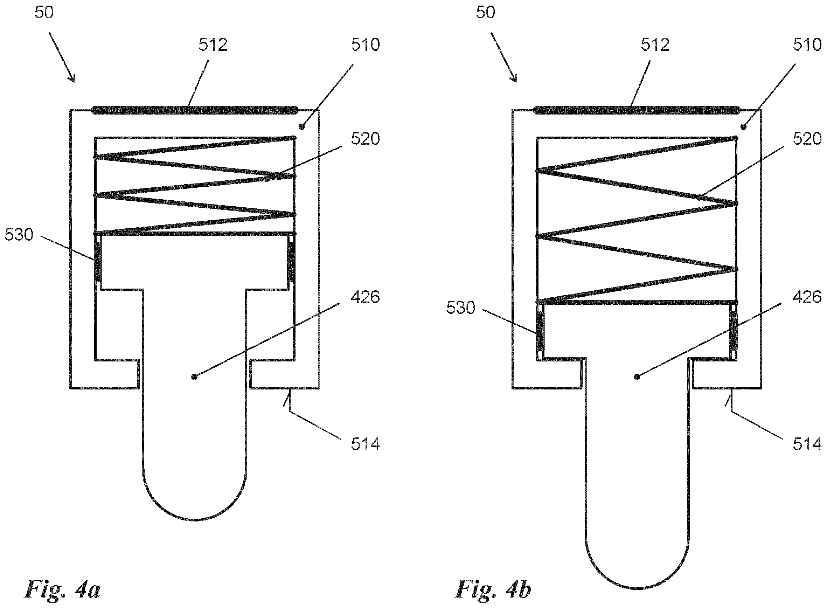

Die Relativlage von funktionseinheitsseitiger Platine 410 und gehäuseseitiger Platine 420 ist in Figur 3 detailliert dargestellt. Bei der gezeigten Ausführungsform beträgt die funktionseinheitsseitige Platine 410 drei konzentrische Ringkontakte 412. Die gehäuseseitige Platine 420 trägt drei korrespondierende Lötstellen 512, an denen je eine Kontakt-Baueinheit 50, wie sie schematisch in Figur 4 dargestellt sind, fixiert ist. Auf die besondere Gestaltung der Kontakt-Baueinheiten 50 soll weiter unten noch näher eingegangen werden. An dieser Stelle genügt es zu verstehen, dass mit jeder der Lötstellen 512 ein axial beweglich gelagerter und in Richtung auf die funktionseinheitsseitige Platine 410 federvorgespannter Kontaktstift 426 elektrisch verbunden ist. Die Lötstellen 512 bzw. die entsprechenden Kontaktstifte 426 sind dabei so angeordnet, dass je ein Kontaktstift 426 einen der Ringkontakt 412 kontaktiert. Dieser Kontakt bleibt auch bei einer konzentrischen Relativdrehung der Platinen 410, 420 erhalten, wobei aufgrund der federelastisch vorgespannten Lagerung der Kontaktstifte 426 dabei auch axiale Abstandsvariationen zwischen den Platinen 410, 420, wie sie beispielsweise durch Fertigungstoleranzen und/oder nicht ideal parallele Positionierung der Platinen 410, 420 zustande kommen können, ausgeglichen werden.

Mit anderen Worten stellt die Schnittstelle 40 also eine elektrische Verbindung zwischen den Schaltern 424a,b des Handstücks 30 und dem Steuerstrang des Versorgungs- und Steuerkabels 230 der Funktionseinheit 20 dar, die auch dann erhalten bleibt, wenn sich die relative Drehlage zwischen der Funktionseinheit 20 und dem Handstück 30, insbesondere durch Betätigung des Drehrades 322, ändert. Der Chirurg kann also bei seiner Arbeit stets eine optimale Handhaltung bewahren, in der er zur Erzeugung von Steuerbefehlen für die Energieversorgung des Ultraschallwandlers 210 in der Lage ist, und gleichzeitig die Funktionseinheit 20, insbesondere das chirurgische Instrument an deren distalem Ende, in die für den konkreten Eingriff erforderliche Drehlage überführen kann.

Figur 4 zeigt grob schematisch eine mögliche Ausgestaltung der Kontakt-Baueinheit 50. Bei der dargestellten Ausführungsform umfasst diese ein Kontaktgehäuse 510, in welchem ein als der Kontaktstift 426 wirkender Stößel axial beweglich gelagert ist. Der Stößel 426 durchsetzt die der funktionseinheitsseitigen Platine 410 zugewandte axiale Stirnseite 514 des Kontaktgehäuses 510 und ist in dieser Richtung im Inneren des Gehäuses durch eine Feder 520 vorgespannt. Ein elektrischer Kontakt zwischen dem Kontaktstift/Stößel 426 und dem Kontaktgehäuse 510 kann über eine entsprechende Ausgestaltung der Feder 520 oder durch direkten Kontakt zwischen Kontaktgehäuse 510 und Kontaktstift/Stößel 426 erfolgen. In Figur 4 wird dieser elektrische Kontakt rein funktional durch einen den Kontaktstift/Stößel 426 ringförmig umlaufenden Schleifring 530 angedeutet. Die rückwärtige Stirnseite des Kontaktgehäuses 510 bildet die Kontaktfläche zur gehäuseseitigen Platine 420 und entspricht im Montageendzustand der Lötstelle 512.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben. Insbesondere kann die axial bewegliche, federvorgespannte Lagerung der Kontaktstifte 426 auch auf andere als die dargestellte Weise realisiert sein. Beispielsweise können die Kontaktstifte 426 direkt in entsprechenden Kanälen des Platinenadapters 430 gelagert sein. Derartige Kanäle können auch im Fall des Einsatzes von Kontakt-Baueinheiten 50 sinnvoll sein, nämlich beispielsweise zur Positionierung derselben beim Lötvorgang und/oder zur seitlichen elektrischen Isolierung.

### Bezugszeichenliste

- 10: Chirurgisches Ultraschallgerät
- 20: Funktionseinheit
- 210: Ultraschallwandler
- 212: Raststutzen
- 220: Schaft
- 230: Versorgungs- und Steuerkabel
- 30: Handstück
- 310: Griffstück
- 312a,b: Drucktaste
- 314: Hebel
- 320: Einsatzstück
- 322: Drehrad
- 330: Funktionseinheits-Adapter
- 332: Rasthaken
- 40: Schnittstelle
- 410: funktionseinheitsseitige Platine
- 412: Ringkontakt
- 414: Anschlusskabel
- 416: Rastkontakt
- 420: gehäuseseitige Platine
- 422: flexible Leiterbahn
- 424a,b: Schalter
- 426: Kontaktstift / Stößel
- 430: Platinenadapter
- 432: Rastnase
- 434: Rastnase
- 50: Kontakt-Baueinheit
- 510: Gehäuse
- 512: Lötstelle
- 514: Stirnwand
- 520: Feder
- 530: Schleifring

## Patentansprüche

1. Handstück (30) für ein chirurgisches Ultraschallgerät (10), umfassend ein Gehäuse, in welches eine Funktionseinheit (20), die einen Ultraschallwandler (210) und ein ultraschallübertragend mit diesem verbundenes Instrument aufweist, derart einsetzbar ist, dass sie das Gehäuse entlang einer Längsachse durchsetzt und um die Längsachse rotierbar in dem Gehäuse gelagert und über eine Steuerleitung mit einem Schalter (424a,b) des Gehäuses elektrisch verbunden ist,
wobei die Steuerleitung eine drehentkoppelnde, elektrische Schnittstelle (40) aufweist, umfassend eine gehäuseseitige Platine (420) und eine funktionseinheitsseitige Platine (410), von denen die eine einen zur Längsachse koaxialen Ringkontakt (412) und die andere einen den Ringkontakt (412) elastisch kontaktierenden, axial erstreckten Kontaktstift (426) trägt,
**dadurch gekennzeichnet,**
**dass** der Kontaktstift (426) als steifer Metallstift ausgebildet ist, der axial beweglich gelagert und in Richtung auf den Ringkontakt (412) federvorgespannt ist.

2. Handstück (30) nach Anspruch 1,
**dadurch gekennzeichnet,**
**dass** die funktionseinheitsseitige Platine (410) an einem um die Längsachse rotierbar im Gehäuse gelagerten Funktionseinheits-Adapter (330) fixiert ist, in welchen die Funktionseinheit (20) reversibel, drehfest und mit elektrischem Kontakt zu der funktionseinheitsseitigen Platine (410) einsetzbar ist.

3. Handstück (30) nach Anspruch 2,
**dadurch gekennzeichnet,**
**dass** der Funktionseinheits-Adapter (330) wenigstens einen mit der funktionseinheitsseitigen Platine (410) elektrisch verbundenen Rastkontakt (416) aufweist, der räumlich mit einem Gegenkontakt der Funktionseinheit (20) derart korrespondiert, dass der Rastkontakt (416) und der Gegenkontakt im eingesetzten Zustand der Funktionseinheit (20) einander elektrisch kontaktieren.

4. Handstück (30) nach einem der vorangehenden Ansprüche,
**dadurch gekennzeichnet,**
**dass** die gehäuseseitige Platine (420) formschlüssig in einem im Gehäuse fixierten Platinenadapter (430) fixiert ist.

5. Handstück (30) nach Anspruch 4,
**dadurch gekennzeichnet,**
**dass** der Platinenadapter (430) formschlüssig im Gehäuse fixiert ist.

6. Handstück (30) nach einem der vorangehenden Ansprüche,
**dadurch gekennzeichnet,**
**dass** der Kontaktstift (426) auf der gehäuseseitigen Platine (420) angeordnet ist.

7. Handstück (30) nach Anspruch 6, soweit rückbezogen auf Anspruch 4,
**dadurch gekennzeichnet,**
**dass** der Kontaktstift (426) in einem achsparallel zur Längsachse erstreckten Kanal des aus einem elektrisch isolierenden Material gefertigten Platinenadapters (430) angeordnet ist.

8. Handstück (30) nach einem der vorangehenden Ansprüche,
**dadurch gekennzeichnet,**
**dass** der Kontaktstift (426) Bestandteil einer Kontakt-Baueinheit (50) ist, die ein elektrisch mit der zugeordneten Platine (420) verbundenes Kontaktgehäuse (510) und einen eine axiale Stirnwand (514) des Kontaktgehäuses (510) durchsetzenden, innerhalb des Kontaktgehäuses (510) mittels einer Feder (520) vorgespannten und als der Kontaktstift (426) wirkenden Stößel umfasst, wobei zumindest das Kontaktgehäuse (50) und der Stößel aus einem elektrisch leitfähigen Material gefertigt sind.

9. Handstück (30) nach einem der vorangehenden Ansprüche,
**dadurch gekennzeichnet,**
**dass** das Gehäuse pistolengriffartig mit einem den Schalter (424a,b) tragenden Griffstück (310) und einem endständig an dem Griffstück (310) abgewinkelt dazu angeordneten Einsatzstück (320) zur Aufnahme der Funktionseinheit (20) ausgebildet ist.

10. Handstück (30) nach einem der vorangehenden Ansprüche,
**dadurch gekennzeichnet,**
**dass** der Schalter (424a,b) mehrere Schaltelemente und/oder das Handstück (30) mehrere Schalter (424a,b) aufweist und die drehentkoppelnde, elektrische Schnittstelle (40) eine entsprechende Mehrzahl von Paaren aus je einem Ringkontakt (412) und einem oder mehreren korrespondierenden Kontaktstiften (426) aufweist, wobei jedes der Paare mit einem anderen oder einer anderen Kombination der Schalter (424a,b) oder Schaltelemente elektrisch verbunden ist.

11. Chirurgisches Ultraschallgerät (10), umfassend
- eine Funktionseinheit (20), die einen Ultraschallwandler (210) und ein ultraschallübertragend mit diesem verbundenes Instrument sowie ein mit einer externen Versorgungs- und Steuereinheit verbindbares Versorgungs- und Steuerkabel (230) aufweist,
- ein Handstück (30) mit einem Gehäuse, in welches die Funktionseinheit (20) derart eingesetzt ist, dass sie das Gehäuse entlang einer Längsachse durchsetzt und um die Längsachse rotierbar in dem Gehäuse gelagert ist,
wobei ein Steuerstrang des Versorgungs- und Steuerkabels (230) über eine Steuerleitung mit einem Schalter (424a,b) des Gehäuses elektrisch verbunden ist, und die Steuerleitung eine drehentkoppelnde, elektrische Schnittstelle (40) aufweist, umfassend eine gehäuseseitige Platine (420) und eine funktionseinheitsseitige Platine (410), von denen die eine einen zur Längsachse koaxialen Ringkontakt (412) und die andere einen den Ringkontakt (412) elastisch kontaktierenden, axial erstreckten Kontaktstift (426) trägt,
**dadurch gekennzeichnet,**
**dass** der Kontaktstift (426) als steifer Metallstift ausgebildet ist, der axial beweglich gelagert und in Richtung auf den Ringkontakt (412) federvorgespannt ist.

12. Chirurgisches Ultraschallgerät (10) nach Anspruch 11,
**dadurch gekennzeichnet,**
**dass** die funktionseinheitsseitige Platine (410) an einem um die Längsachse rotierbar im Gehäuse gelagerten Funktionseinheits-Adapter (330) fixiert ist, in welchem die Funktionseinheit (20) reversibel, drehfest und mit elektrischem Kontakt zu der funktionseinheitsseitigen Platine (410) fixiert ist.

13. Chirurgisches Ultraschallgerät (10) nach Anspruch 12,
**dadurch gekennzeichnet,**
**dass** der Funktionseinheits-Adapter (330) wenigstens einen mit der funktionseinheitsseitigen Platine (410) elektrisch verbundenen Rastkontakt (416) aufweist, der räumlich mit einem Gegenkontakt der Funktionseinheit (20) derart korrespondiert, dass der Rastkontakt (416) und der Gegenkontakt einander elektrisch kontaktieren.

14. Chirurgisches Ultraschallgerät (10) einem der Ansprüche 11 bis 13,
**dadurch gekennzeichnet,**
**dass** ein Versorgungsstrang des Versorgungs- und Steuerkabels (230) mit einer vibrationserzeugenden Einheit des Ultraschallwandler (210) elektrisch verbunden ist.

## Claims

1. A handpiece (30) for an ultrasonic surgical apparatus (10) comprising a housing into which is insertable a functional unit (20) having an ultrasonic transducer (210) and an instrument connected thereto in an ultrasound transmitting manner, said functional unit (20) is insertable so as to pass through the housing along a longitudinal axis and to be rotatably mounted in the housing about the longitudinal axis and electrically connected to a switch (424a,b) of the housing via a control line, wherein the control line has a rotationally decoupling electrical interface (40) comprising a housing-side circuit board (420) and a functional unit-side circuit board (410), one of which carries a ring contact (412) coaxial with the longitudinal axis and the other of which carries an axially extended contact pin (426) elastically contacting the ring contact (412),
**characterised**
**in that** the contact pin (426) is designed as a rigid metal pin which is mounted axially movably and is spring-biased in the direction of the ring contact (412).

2. The handpiece (30) according to claim 1,
**characterised in that**
the functional unit-side circuit board (410) is fixed to a functional unit adapter (330) which is mounted in the housing such that it can rotate about the longitudinal axis and into which the functional unit (20) can be inserted in a reversible, rotationally fixed manner and with electrical contact to the functional unit-side circuit board (410).

3. The handpiece (30) according to claim 2,
**characterised**
**in that** the functional unit adapter (330) has at least one latching contact (416) which is electrically connected to the functional unit-side circuit board (410) and which spatially corresponds to a mating contact of the functional unit (20) in such a way that the latching contact (416) and the mating contact make electrical contact with one another when the functional unit (20) is in the inserted state.

4. The handpiece (30) according to one of the preceding claims,
**characterised**
**in that** the housing-side circuit board (420) is positively fixed in a circuit board adapter (430) fixed in the housing.

5. The handpiece (30) according to claim 4,
**characterised**
**in that** the circuit board adapter (430) is positively fixed in the housing.

6. The handpiece (30) according to one of the preceding claims,
**characterised**
**in that** the contact pin (426) is arranged on the housing-side circuit board (420).

7. The handpiece (30) according to claim 6, as far as referred back to claim 4,
**characterised**
**in that** the contact pin (426) is arranged in a channel, extending axially parallel to the longitudinal axis, of the circuit board adapter (430) made of an electrically insulating material.

8. The handpiece (30) according to one of the preceding claims,
**characterised**
**in that** the contact pin (426) is a component of a contact assembly (50) comprising a contact housing (510) electrically connected to the associated circuit board (420) and a plunger passing through an axial end wall (514) of the contact housing (510), biased within the contact housing (510) by means of a spring (520) and acting as the contact pin (426), wherein at least the contact housing (50) and the plunger are made of an electrically conductive material.

9. The handpiece (30) according to one of the preceding claims,
**characterised**
**in that** the housing is formed in the manner of a pistol grip with a grip piece (310) carrying the switch (424a,b) and an insert piece (320) arranged at the end of the grip piece (310) at an angle thereto for receiving the functional unit (20).

10. The handpiece (30) according to any one of the preceding claims,
**characterised**
**in that** the switch (424a,b) comprises a plurality of switching elements and/or the handpiece (30) comprises a plurality of switches (424a,b) and the rotationally decoupling electrical interface (40) comprises a corresponding plurality of pairs each comprising a ring contact (412) and one or more corresponding contact pins (426), each of the pairs being electrically connected to a different one or a different combination of the switches (424a,b) or switching elements.

11. An ultrasonic surgical apparatus (10) comprising
- a functional unit (20) comprising an ultrasonic transducer (210) and an instrument connected thereto in an ultrasound transmitting manner, and a supply and control cable (230) connectable to an external supply and control unit,
- a handpiece (30) with a housing in which the functional unit (20) is inserted in such a way that it passes through the housing along a longitudinal axis and is rotatably mounted in the housing about the longitudinal axis,
wherein a control strand of the supply and control cable (230) is electrically connected to a switch (424a,b) of the housing via a control line, and the control line has a rotationally decoupling electrical interface (40), comprising a housing-side circuit board (420) and a functional unit-side circuit board (410), one of which carries a ring contact (412) coaxial with the longitudinal axis and the other of which carries an axially extended contact pin (426) elastically contacting the ring contact (412) ,
**characterised**
**in that** the contact pin (426) is designed as a rigid metal pin which is mounted axially movably and spring-biased in the direction of the ring contact (412).

12. The ultrasonic surgical apparatus (10) according to claim 11,
**characterized**
**in that** the functional unit-side circuit board (410) is fixed to a functional unit adapter (330) which is mounted in the housing such that it can rotate about the longitudinal axis and in which the functional unit (20) is fixed in a reversible, rotationally fixed manner and with electrical contact to the functional unit-side circuit board (410).

13. The ultrasonic surgical apparatus (10) according to claim 12,
**characterised**
**in that** the functional unit adapter (330) has at least one latching contact (416) electrically connected to the functional unit-side circuit board (410), the latching contact (416) corresponding spatially to a mating contact of the functional unit (20) such that the latching contact (416) and the mating contact make electrical contact with each other.

14. The ultrasonic surgical apparatus (10) according to any one of claims 11 to 13, **characterised**
**in that** a supply strand of the supply and control cable (230) is electrically connected to a vibration generating unit of the ultrasonic transducer (210).

## Revendications

1. Une pièce à main (30) pour un appareil chirurgical à ultrasons (10), comprenant un boîtier dans lequel une unité fonctionnelle (20), qui présente un transducteur à ultrasons (210) et un instrument connecté à celui-ci d'une manière capable de transmettre des ultrasons, peut être insérée de telle sorte qu'elle traverse le boîtier le long d'un axe longitudinal et est montée dans le boîtier de manière à pouvoir tourner autour de l'axe longitudinal et est connectée électriquement à un commutateur (424a, b) du boîtier par l'intermédiaire d'une ligne de commande,
la ligne de commande présentant une interface électrique (40) de découplage en rotation, comprenant une platine (420) côté boîtier et une platine (410) côté unité fonctionnelle, dont l'une porte un contact annulaire (412) coaxial à l'axe longitudinal et l'autre une tige de contact (426) s'étendant axialement, en contact élastique avec le contact annulaire (412),
**caractérisé en ce**
**que** la tige de contact (426) est réalisée sous la forme d'une tige métallique rigide, qui est montée mobile axialement et est précontrainte par ressort en direction du contact annulaire (412).

2. La pièce à main (30) selon la revendication 1,
**caractérisée en ce**
**que** la platine (410) côté unité fonctionnelle est fixée à un adaptateur (330) d'unité fonctionnelle monté dans le boîtier de manière à pouvoir tourner autour de l'axe longitudinal, dans lequel l'unité fonctionnelle (20) peut être insérée de manière réversible, sans pouvoir tourner et avec un contact électrique avec la platine (410) côté unité fonctionnelle.

3. La pièce à main (30) selon la revendication 2,
**caractérisé en ce**
**que** l'adaptateur d'unité fonctionnelle (330) présente au moins un contact d'encliquetage (416) relié électriquement à la platine (410) côté unité fonctionnelle, qui correspond spatialement à un contact opposé de l'unité fonctionnelle (20) de telle sorte que le contact d'encliquetage (416) et le contact opposé entrent en contact électrique l'un avec l'autre lorsque l'unité fonctionnelle (20) est en place.

4. La pièce à main (30) selon l'une des revendications précédentes,
**caractérisé en ce**
**que** la platine (420) côté boîtier est fixée par complémentarité de forme dans un adaptateur de platine (430) fixé dans le boîtier.

5. La pièce à main (30) selon la revendication 4,
**caractérisé en ce**
**que** l'adaptateur de platine (430) est fixé par complémentarité de forme dans le boîtier.

6. La pièce à main (30) selon l'une des revendications précédentes,
**caractérisé en ce**
**que** la tige de contact (426) est disposée sur la platine (420) côté boîtier.

7. La pièce à main (30) selon la revendication 6, dans la mesure où elle se rapporte à la revendication 4,
**caractérisé en ce**
**que** la tige de contact (426) est disposée dans un canal de l'adaptateur de platine (430) fabriqué en un matériau électriquement isolant, qui s'étend parallèlement à l'axe longitudinal.

8. La pièce à main (30) selon l'une des revendications précédentes,
**caractérisé en ce**
**que** la tige de contact (426) fait partie d'un ensemble de contact (50) qui comprend un boîtier de contact (510) connecté électriquement à la platine (420) associée et un poussoir traversant une paroi frontale axiale (514) du boîtier de contact (510), précontraint à l'intérieur du boîtier de contact (510) au moyen d'un ressort (520) et agissant comme la tige de contact (426), au moins le boîtier de contact (50) et le poussoir étant fabriqués en un matériau électriquement conducteur.

9. La pièce à main (30) selon l'une des revendications précédentes,
**caractérisé en ce**
**que** le boîtier est réalisé à la manière d'une poignée de pistolet avec une pièce de préhension (310) portant le commutateur (424a, b) et une pièce d'insertion (320) disposée à l'extrémité sur la pièce de préhension (310) et coudée par rapport à celle-ci pour recevoir l'unité fonctionnelle (20).

10. La pièce à main (30) selon l'une quelconque des revendications précédentes, **caractérisée en ce**
**que** le commutateur (424a, b) comprend plusieurs éléments de commutation et/ou la pièce à main (30) comprend plusieurs commutateurs (424a, b) et l'interface électrique de découplage en rotation (40) comprend une pluralité correspondante de paires constituées chacune d'un contact annulaire (412) et d'une ou plusieurs tiges de contact correspondantes (426), chacune des paires étant connectée électriquement à un commutateur différent ou à une combinaison différente des commutateurs (424a, b) ou des éléments de commutation.

11. Un Appareil chirurgical à ultrasons (10), comprenant
- une unité fonctionnelle (20) qui présente un transducteur à ultrasons (210) et un instrument connecté à celui-ci d'une manière capable de transmettre des ultrasons ainsi qu'un câble d'alimentation et de commande (230) pouvant être connecté à une unité d'alimentation et de commande externe,
- une pièce à main (30) avec un boîtier dans lequel l'unité fonctionnelle (20) est insérée de telle sorte qu'elle traverse le boîtier le long d'un axe longitudinal et est logée dans le boîtier de manière à pouvoir tourner autour de l'axe longitudinal,
un brin de commande du câble d'alimentation et de commande (230) étant relié électriquement à un commutateur (424a, b) du boîtier par l'intermédiaire d'une ligne de commande, et la ligne de commande présentant une interface électrique (40) à découplage en rotation, comprenant une platine côté boîtier (420) et une platine côté unité fonctionnelle (410), dont l'une porte un contact annulaire (412) coaxial à l'axe longitudinal et l'autre une tige de contact (426) s'étendant axialement et contactant élastiquement le contact annulaire (412) ,
**caractérisé en ce**
**que** la tige de contact (426) est réalisée sous la forme d'une tige métallique rigide, qui est montée mobile axialement et est précontrainte par ressort en direction du contact annulaire (412).

12. L'appareil chirurgical à ultrasons (10) selon la revendication 11,
**caractérisé en ce**
**que** la platine (410) côté unité fonctionnelle est fixée à un adaptateur (330) d'unité fonctionnelle monté dans le boîtier de manière à pouvoir tourner autour de l'axe longitudinal, dans lequel l'unité fonctionnelle (20) est fixée de manière réversible, sans pouvoir tourner et avec un contact électrique avec la platine (410) côté unité fonctionnelle.

13. L'appareil chirurgical à ultrasons (10) selon la revendication 12,
**caractérisé en ce**
**que** l'adaptateur d'unité fonctionnelle (330) présente au moins un contact d'encliquetage (416) relié électriquement à la platine (410) côté unité fonctionnelle, qui correspond spatialement à un contact opposé de l'unité fonctionnelle (20) de telle sorte que le contact d'encliquetage (416) et le contact opposé se contactent électriquement.

14. L'appareil chirurgical à ultrasons (10) selon l'une des revendications 11 à 13, **caractérisé en ce**
**qu'**un brin d'alimentation du câble d'alimentation et de commande (230) est connecté électriquement à une unité de génération de vibrations du transducteur à ultrasons (210).
